# FASCICULE DE BREVET EUROPEEN

(11) **EP 1 106 595 B1**
(45) Date de publication et mention de la délivrance du brevet: **11.08.2004**
(21) Numéro de dépôt: 00403041.7
(22) Date de dépôt: 02.11.2000
(51) Int. Cl.: C07C 45/50, C07C 47/02

(54) **Procédé amélioré d'hydroformylation au moyen d'un catalyseur à base de cobalt et/ou de rhodium mis en oeuvre en milieu biphasique**
Verbessertes Hydroformylierungsverfahren mit Katalysatoren auf der Basis von Kobalt und/oder Rhodium in einem biphasischen Medium
Improved hydroformylation process with cobalt and/or rhodium based catalysts in a biphasic medium

(30) Priorité: 08.12.1999 FR 9915573
(43) Date de publication de la demande: 13.06.2001
(73) Titulaire: Institut Français du Pétrole, 92852 Rueil-Malmaison Cedex (FR)
(72) Inventeur: Hillebrand, Gerhard, 92500 Rueil Maimaison (FR); Hirschauer, André, 78360 Montesson (FR); Commereuc, Dominique, 92190 Meudon (FR); Olivier-Bourbigou, Hélène, 92500 Rueil Malmaison (FR); Saussine, Lucien, 78290 Croissy sur Seine (FR)

(56) Documents cités:
- EP-A- 0 107 430
- EP-A- 0 776 880
- EP-A- 0 924 182

## Description

La présente invention concerne un procédé amélioré d'hydroformylation de composés oléfiniquement insaturés au moyen d'un catalyseur à base de cobalt et/ou de rhodium mis en oeuvre en milieu biphasique. Le catalyseur à base de cobalt et/ou de rhodium, qui comprend au moins un complexe du cobalt et/ou du rhodium coordiné par au moins un ligand azoté, est dissous dans un solvant ionique non-aqueux, liquide à une température inférieure à 90 °C, dans lequel les aldéhydes formés sont peu ou pas solubles.

L'hydroformylation des composés oléfiniques est une réaction de grande importance industrielle et la plupart des procédés ont recours à des catalyseurs homogènes dissous dans une phase organique constituée des réactifs, des produits et éventuellement d'un excès de ligand, si bien que l'on rencontre des difficultés pour séparer et récupérer le catalyseur, en particulier quand celui-ci est employé en relativement grande quantité, comme c'est le cas avec les catalyseurs à base de cobalt, ou quand celui-ci est un métal noble, comme c'est le cas avec les catalyseurs à base de rhodium.

Une solution visant à résoudre ce problème a été évoquée par Bartik et al. : Organometallics (1993) 12 164-170, J. Organometal. Chem. (1994) 480 15-21, ainsi que par Beller et al.: J. molecular Catal. A: Chemical (1999) 143 31-39. Elle consiste à effectuer l'hydroformylation en présence d'une solution aqueuse contenant un complexe du cobalt qui est rendu hydrosoluble grâce à la présence d'un ligand phosphine-sulfonate tel que le sel de sodium de la triphénylphosphine trisulfonée ou d'une tris-(alkylphényl)-phosphine trisulfonée. La demande de brevet internationale WO-A-97/00132 décrit des clusters de cobalt substitués par des groupements trialkoxysilylméthyle qui les rendent solubles dans l'eau. De cette manière la phase organique contenant les aldéhydes est aisément séparée de la phase aqueuse contenant le catalyseur.

Une autre solution visant à résoudre ce problème a été décrite dans le document de brevet français FR-A-2 314 910. Elle consiste à effectuer l'hydroformylation en présence d'une solution aqueuse contenant un complexe du rhodium qui est rendu hydrosoluble grâce à la présence d'un ligand phosphine sulfonée lui-même hydrosoluble, tel que le sel de sodium de la triphénylphosphine trisulfonée. De cette manière, la phase organique contenant les aldéhydes est aisément séparée de la phase aqueuse contenant le catalyseur. Cette technique a fait l'objet d'un nombre considérable de travaux, qui ont été discutés dans un article de W.A. Herrmann paru dans « Angewandte Chemie International » en 1993, volume 32, page 1524 et suivantes.

En dépit du grand intérêt industriel de ces techniques pour l'hydroformylation du propylène, ces systèmes à deux phases souffrent du manque de solubilité des oléfines dans l'eau, ce qui conduit à des vitesses de réaction relativement faibles, qui les rendent inapplicables pour les oléfines à longue chaîne.

Par ailleurs a été décrit dans le brevet US-A-3 565 823 une technique consistant à disperser un composé d'un métal de transition dans un sel d'étain ou de germanium et d'un ammonium ou d'un phosphonium quaternaire, de formule (R¹R²R³R⁴Z)YX₃ dans laquelle R¹, R², R³ et R⁴, sont des restes hydrocarbyles ayant jusqu'à 18 atomes de carbone, Z est l'azote ou le phosphore, Y l'étain ou le germanium et X un halogène, par exemple le chlore ou le brome.

Dans le brevet US-A-3 832 391 a été revendiqué un procédé de carbonylation des oléfines par la même composition. Les compositions précédentes présentent le désavantage d'avoir un point de fusion relativement élevé, par exemple supérieur à 90 °C, ce qui complique la manipulation des solutions de catalyseur et des produits de réaction.

Il a été décrit dans le brevet US-A-5 874 638 au nom de la demanderesse que l'on peut à la fois bénéficier des avantages d'une mise en oeuvre à deux phases tout en évitant les inconvénients liés d'une part à l'utilisation de l'eau et d'autre part à l'emploi de composés à point de fusion élevé, en dissolvant certains composés catalytiques des métaux de transition des groupes 8, 9 et 10, connus pour catalyser l'hydroformylation, dans des solvants ioniques non-aqueux qui sont constitués par des sels organiques-inorganiques liquides à température ambiante.

On connaît le document EP-A-0 924 182, qui divulgue un procédé d'hydroformylation d'oléfines en phase liquide en présence d'un catalyseur au rhodium et d'un liquide ionique non-aqueux de formule (Q⁺)ₐ A^{a-}. L'anion A^{a-} est défini comme étant essentiellement une triarylphosphine sulfonée. Q⁺ est un cation ammonium ou phosphonium quaternaire. Il est mentionné dans ce document que notamment l'amine dérivée de Q⁺ peut être en excès par rapport à la stoechiométrie de (Q⁺)ₐ A^{a-}.

Il a maintenant été trouvé que l'activité et la sélectivité, pour la réaction d'hydroformylation, des catalyseurs à base de cobalt et/ou de rhodium mis en oeuvre dans un solvant ionique non-aqueux, liquide à une température inférieure à 90 °C, sont grandement améliorées par l'utilisation de ligands azotés pour complexer le cobalt et/ou de rhodium.

Plus précisément, l'invention a pour objet un procédé pour l'hydroformylation en phase liquide des composés oléfiniquement insaturés dans lequel la réaction est effectuée en présence d'une composition catalytique choisie parmi :
- celles qui comprennent au moins un complexe du cobalt coordiné par au moins un ligand azoté et au moins un solvant ionique non-aqueux comprenant au moins un sel organique-inorganique répondant à la formule générale Q+A-,
dans laquelle Q+ représente un cation ammonium quaternaire et/ou un cation phosphonium quaternaire, et A- représente tout anion susceptible de former un sel liquide en dessous de 90°C ; et
- celles qui comprennent au moins un complexe du rhodium coordiné par au moins un ligand azoté choisi dans le groupe formé par les imines, les diimines, la pyridine et les pyridines substituées, la bipyridine, imidazole et les imidazoles substitués, le pyrrole et les pyrroles substitués, le pyrazole et les pyrazoles substitués et au moins un solvant ionique non-aqueux comprenant au moins un sel organique-inorganique de formule générale Q⁺A⁻, dans laquelle Q⁺ représente un ammonium quaternaire et/ou un phosphonium quaternaire, et A⁻ représente tout anion susceptible de former un sel liquide en dessous de 90°C.

Les composés du cobalt et/ou du rhodium précurseurs du catalyseur sont choisis dans le groupe formé par les sels de cobalt et/ou de rhodium, comme les acétylacétonates, les carboxylates, en particulier le formiate ou l'acétate, et les complexes carbonyles, comme le dicobalt-octacarbonyle, l'hydrure de cobalt-tétracarbonyle, l'acétylacétonate de rhodium-dicarbonyle et les clusters carbonyles. Le choix du composé précurseur du cobalt et/ou du rhodium n'est pas critique, mais on préfère en général éviter les halogénures.

Le ligand azoté est choisi de préférence dans le groupe formé par les mono-amines, les di-, tri- et polyamines, les imines, les diimines, la pyridine et les pyridines substituées, la bipyridine, l'imidazole et les imidazoles substitués, le pyrrole et les pyrroles substitués, le pyrazole et les pyrazoles substitués. On peut citer à titre d'exemples non limitatifs la triéthylamine, l'éthylènediamine, la tétraméthyl-éthylènediamine, la diéthylènetriamine, le diazabicyclooctane, le 1,4,7-triméthyl-1,4,7-triazacyclononane, la N,N'-diméthyl-éthane-1,2-diimine, la N,N'-di-t-butyl-éthane-1,2-diimine, la N,N'-di-t-butyl-butane-2,3-diimine, la N,N'-diphényl-éthane-1,2-diimine, la N,N'-bis-(diméthyl-2,6-phényl)-éthane-1,2-diimine, la N,N'-bis-(diisopropyl-2,6-phényl)-éthane-1,2-diimine, la N,N'-bis-(di-t-butyl-2,6-phényl)-éthane-1,2-diimine, la N,N'-diphényl-butane-2,3-diimine, la N,N'-bis-(diméthyl-2,6-phényl)-butane-2,3-diimine, la N,N' - bis-(diisopropyl-2,6-phényl) - butane-2,3-diimine, la N,N'-bis-(di-t-butyl-2,6-phényl)-butane-2,3-diimine, la pyridine, les picolines, la t-butylpyridine, la bipyridine, la di-t-butyl-bipyridine, l'imidazole, le N-méthylimidazole, le N-butylimidazole, le benzimidazole, le pyrrole, le N-méthylpyrrole et le 2,6-diméthylpyrrole.

Le ligand azoté peut comporter aussi d'autres fonctions organiques, telles que par exemple des fonctions alcool, aldéhyde, cétone, acide carboxylique, ester, nitrile, ammonium et/ou phosphonium quaternaire, ainsi que sulfonium. On peut citer à titre d'exemples non limitatifs les acides et esters picoliniques, les 2,6-dialkoxypyridines, les salicylaldimines, les 2,6-bis-N-aryliminopyridines, les 1-dialkyl (et diaryl) phosphino-2-(4-pyridyl)-éthanes, les (pyridyl-4)-2-acétates d'alkyle, les (pyridyl-2)-2-acétates d'alkyle, le bis-(pyridyl-4)-3-propanoate d'éthylèneglycol, le (pyridyl-2)-2-éthanol, le (pyridyl-2)-3-propanol et le (pyridyl-2)-3-propyl acétate.

Le solvant ionique non-aqueux est choisi dans le groupe formé par les sels liquides qui ont pour formule générale Q⁺ A⁻ dans laquelle Q⁺ représente un ammonium quaternaire et/ou d'un phosphonium quaternaire et A⁻ représente tout anion susceptible de former un sel liquide à basse température, c'est-à-dire au-dessous de 90 °C et avantageusement d'au plus 85 °C, et de préférence au-dessous de 50 °C. Les anions A⁻ préférés sont les ions acétate, halogéno-acétates, tétrafluoro-borate, tétrachoro-borate, hexafluoro-phosphate, hexafluoro-antimonate, fluoro-sulfonate, perfluoroalkyl-sulfonates, bis-(perfluoroalkyl-sulfonyl)-amidures, arène-sulfonates.

Les ammonium et/ou phosphonium quaternaires répondent de préférence aux formules générales NR¹R²R³R⁴⁺ et PR¹R²R³R⁴⁺ ou aux formules générales R¹R²N = CR³R⁴⁺ et R¹R²P = CR³R⁴⁺, où R¹, R², R3 et R⁴, identiques ou différents, représentent l'hydrogène (à l'exception du cation NH₄⁺ pour NR¹R²R³R⁴⁺), de préférence un seul substituant représentant l'hydrogène, ou des restes hydrocarbyles ayant de 1 à 12 atomes de carbone, par exemple des groupements alkyles, saturés ou non saturés, cycloalkyles ou aromatiques, aryle ou aralkyle, comprenant de 1 à 12 atomes de carbone. Les ammonium et/ou phosphonium peuvent également être dérivés d'hétérocycles azotés et/ou phosphorés comportant 1, 2 ou 3 atomes d'azote et/ou de phosphore, dans lesquelles les cycles sont constitués de 4 à 10 atomes, de préférence 5 à 6 atomes.

L'ammonium ou le phosphonium quaternaire peuvent également être un cation de formule:

R¹R²+N = CR³-R⁵-R³C = N+R¹R²

et/ou

R¹ R²+P = CR³-R⁵-R³C = P+R¹R²

dans laquelle R¹, R² et R³, identiques ou différents sont définis comme précédemment et R⁵ représente un reste alkylène ou phénylène. Parmi les groupements R¹, R², R3 et R⁴, on mentionnera les radicaux méthyle, éthyle, propyle, isopropyle, butyle, secondaire butyle, tertiaire butyle, amyle, méthylène, éthylidène, phényle ou benzyle; R⁵ pourra être un groupement méthylène, éthylène, propylène ou phénylène. Le cation ammonium et/ou phosphonium est choisi de préférence dans le groupe formé par le N-butylpyridinium, le N-éthylpyridinium, l'éthyl-3 méthyl-1 imidazolium, le butyl-3 méthyl-1 imidazolium, le diéthyl-pyrazolium, le pyridinium, le triméthyl-phényl-ammonium et le tétrabutyl-phosphonium. A titre d'exemples des sels utilisables selon l'invention, on peut citer l'hexafluorophosphate de N-butyl-pyridinium, le tétrafluoroborate de N-éthyl- pyridinium, le tétrafluoroborate de tétrabutylphosphonium, le tétrafluoroborate de butyl-3 méthyl-1 imidazolium, l'hexafluoroantimonate de butyl-3 méthyl-1 imidazolium, l'hexafluorophosphate de butyl-3 méthyl-1 imidazolium, le trifluorométhylsulfonate de butyl-3 méthyl-1 imidazolium, le fluorosulfonate de pyridinium et l'hexafluorophosphate de triméthyl-phénylammonium. Ces sels peuvent être utilisés seuls ou en mélange.

La composition catalytique est obtenue par mélange, d'une manière quelconque, du sel liquide avec le composé du cobalt et/ou du rhodium et le ligand azoté. On peut également dissoudre préalablement le composé du métal de transition(cobalt et/ou rhodium) et/ou le ligand dans un solvant organique.

Le complexe entre le précurseur de cobalt et/ou du rhodium et le ligand azoté peut être préparé préalablement à la réaction par mélange du précurseur de cobalt et/ou de rhodium avec le ligand dans un solvant convenable, par exemple un solvant organique ou le solvant ionique non-aqueux qui sera utilisé ensuite dans la réaction catalytique. Le complexe peut aussi être préparé in situ par mélange du précurseur de cobalt et/ou du rhodium et du ligand azoté directement dans le réacteur d'hydroformylation.

D'une façon générale, la composition catalytique peut contenir un solvant organique miscible ou partiellement miscible comme un hydrocarbure aromatique, et/ou un hydrocarbure aliphatique non miscible qui permet une meilleure séparation des phases. De façon préférée, la composition catalytique ne contient pas d'eau.

La concentration du complexe de cobalt et/ou de rhodium dans le solvant ionique liquide "sel fondu" n'est pas critique. Elle est avantageusement comprise entre 0,1 mole par litre de "sel fondu" et 5 moles par litre, de préférence entre 1 mole et 1 mole par litre, et encore entre 100 et 500 moles par litre. Le rapport molaire entre le ligand azoté et le composé du cobalt et/ou du rhodium est compris entre 0,1 et 100, de préférence entre 1 et 20.

Les composants entrant dans la composition selon l'invention peuvent être mélangés dans un ordre quelconque, à une température comprise entre -20 °C et 200 °C, et de préférence entre 0 °C et 140 °C et avantageusement entre 20 °C et 90 °C.

Les composés oléfiniquement insaturés susceptibles d'être hydroformylés sont choisis dans le groupe formé par les monooléfines, les dioléfines et en particulier les dioléfines conjuguées, les composés oléfiniques comportant un ou plusieurs hétéroatomes, notamment insaturés, comme des composés à fonction cétone ou acide carboxylique. A titre d'exemples, on peut citer l'hydroformylation des pentènes en hexanal et methylpentanal, des hexènes en isoheptanals, des isooctènes en isononanals. Ces composés oléfiniques peuvent être mis en oeuvre purs ou dilués par des hydrocarbures saturés ou insaturés.

Le rapport des pressions partielles de l'hydrogène au monoxyde de carbone utilisé dans le milieu réactionnel pour l'hydroformylation peut être de 10:1 à 1:10, de préférence dans un rapport 1:1, mais tout autre rapport peut être utilisé selon la mise en oeuvre du procédé.

La température à laquelle se fera l'hydroformylation sera comprise entre 30 °C et 200 °C, avantageusement la température est inférieure à 150 °C, de préférence entre 50 °C et moins de 150 °C. La pression peut être comprise entre 1 MPa et 20 MPa, de préférence entre 2 MPa et 15 MPa.

La réaction catalytique d'hydroformylation des composés insaturés peut être conduite en système fermé, en système semi-ouvert ou en continu avec un ou plusieurs étages de réaction. A la sortie du réacteur, la phase organique contenant les produits de réaction est séparée avantageusement par simple décantation de la phase solvant ionique contenant le "sel fondu" et la majeure partie du catalyseur. Cette phase solvant ionique, qui contient au moins en partie le catalyseur, est au moins en partie retournée au réacteur, l'autre partie éventuelle étant traitée pour éliminer les résidus du catalyseur.

Les exemples suivants illustrent l'invention sans en limiter la portée.

### EXEMPLE 1

La réaction d'hydroformylation est conduite dans un autoclave en acier inoxydable d'une contenance de 300 mL, muni d'une double enveloppe permettant la régulation de la température par circulation d'un fluide caloporteur, et équipé d'une agitation mécanique efficace avec pales et contre-pales. Dans cet autoclave purgé au préalable de l'air et de l'humidité et placé sous la pression atmosphérique du mélange hydrogène-monoxyde de carbone (1/1 molaire), on introduit 0,4 g de dicobalt-octacarbonyle (soit 2,3 millimoles de cobalt), 0,16 g de pyridine (2 millimoles), 10 mL de tétrafluoro-borate de butyl-méthyl-imidazolium, 30 mL d'heptane et 30 mL d'hexène-1. On porte la pression du mélange hydrogène-monoxyde de carbone (1/1 molaire) à 10 MPa et la température à 125 °C et on met l'agitation en route. Après 6 heures, on arrête l'agitation et on laisse refroidir et décanter le mélange réactionnel, puis on relâche la pression. Après soutirage hors de l'autoclave, la phase organique supérieure est légèrement colorée. La conversion de l'hexène-1 est de 98,9 % en poids. La sélectivité pour les aldéhydes en C7 est de 88,9 % et le rapport n/iso (n-heptanal / isoheptanals) est égal à 1,9.

### EXEMPLE 2

La réaction d'hydroformylation est conduite dans le même appareillage et selon le même mode opératoire que décrit dans l'exemple 1. On introduit 0,4 g de dicobalt-octacarbonyle (2,3 millimoles de cobalt), 0,16 g de pyridine (2 millimoles), 10 mL de tétrafluoro-borate de butyl-méthyl-imidazolium, 30 mL d'heptane et 30 mL d'une coupe d'oléfines en C6 contenant 7,4 % de diméthyl-2,3-butène-1, 12,1 % de diméthyl-2,3-butène-2, 23,5 % de méthyl-2-pentène-1 et 57 % de méthyl-2-pentène-2. On porte la pression du mélange hydrogène-monoxyde de carbone (1/1 molaire) à 12 MPa et la température à 90 °C et on met l'agitation en route. Après 6 heures, on arrête l'agitation et on laisse refroidir et décanter le mélange réactionnel, puis on relâche la pression. Après soutirage hors de l'autoclave, la phase organique supérieure est légèrement colorée. La conversion des oléfines en C6 est de 66 % en poids. La sélectivité pour les aldéhydes en C7 est de 60 %. Les autres produits sont des hexanes (18 %), ainsi que des produits lourds.

### EXEMPLE 3

La réaction d'hydroformylation est conduite dans le même appareillage et selon le même mode opératoire que décrit dans l'exemple 1. On introduit 0,35 g de dicobalt-octacarbonyle (2 millimoles de cobalt), 0,28 g de 1-dicyclopentyl-phosphino-2-(4-pyridyl)-éthane (1 mole), 10 mL de tétrafluoro-borate de butyl-méthyl-imidazolium, 10 mL d'heptane et 30 mL d'hexène-1. On porte la pression du mélange hydrogène-monoxyde de carbone (1/1 molaire) à 11 MPa et la température à 140 °C et on met l'agitation en route. Après 6 heures, on arrête l'agitation et on laisse refroidir et décanter le mélange réactionnel, puis on relâche la pression. Après soutirage hors de l'autoclave, la phase organique supérieure est très légèrement colorée. La conversion de l'hexène-1 est de 83,8 % en poids. La sélectivité pour les aldéhydes en C7 est de 52,8 % et le rapport n/iso (n-heptanal / iso-heptanals) est égal à 1,6. Les autres produits sont des hexènes-2 et -3 (11 %), ainsi que des produits lourds.

### EXEMPLE 4

La réaction d'hydroformylation est conduite dans le même appareillage et selon le même mode opératoire que décrit dans l'exemple 1. On introduit 1,6 g de dicobalt-octacarbonyle (9,3 millimoles de cobalt), 3,3 g de bis-(pyridyl-4)-3-propanoate d'éthylèneglycol (10 millimoles), 10 mL de tétrafluoro-borate de butyl-méthyl-imidazolium, 30 mL d'heptane et 30 mL d'hexène-1. On porte la pression du mélange hydrogène-monoxyde de carbone (1/1 molaire) à 9,5 MPa et la température à 95 °C et on met l'agitation en route. Après 6 heures, on arrête l'agitation et on laisse refroidir et décanter le mélange réactionnel, puis on relâche la pression. Après soutirage hors de l'autoclave, la phase organique supérieure est pratiquement incolore. La conversion de l'hexène-1 est de 65 % en poids. La sélectivité pour les aldéhydes en C7 est de 67 % et le rapport n/iso (n-heptanal / isoheptanals) est égal à 4,2. Les autres produits sont des alcools en C7 (11 %), ainsi que des produits lourds.

### EXEMPLE 5

La réaction d'hydroformylation est conduite dans le même appareillage et selon le même mode opératoire que décrit dans l'exemple 1. On introduit 1,6 g de dicobalt-octacarbonyle (9,3 millimoles de cobalt), 0,8 g de bis-(pyridyl-4)-3-propanoate d'éthylèneglycol (2,4 millimoles), 10 mL de tétrafluoro-borate de butyl-méthyl-imidazolium, 30 mL d'heptane et 30 mL d'hexène-1. On porte la pression du mélange hydrogène-monoxyde de carbone (1/1 molaire) à 8 MPa et la température à 80 °C et on met l'agitation en route. Après 6 heures, on arrête l'agitation et on laisse refroidir et décanter le mélange réactionnel, puis on relâche la pression. Après soutirage hors de l'autoclave, la phase organique supérieure est peu colorée. La conversion de l'hexène-1 est de 68 % en poids. La sélectivité pour les aldéhydes en C7 est de 90 % et le rapport n/iso (n-heptanal / isoheptanals) est égal à 3,9.

### EXEMPLE 6

La réaction d'hydroformylation est conduite dans le même appareillage et selon le même mode opératoire que décrit dans l'exemple 1. On introduit 0,4 g de dicobalt-octacarbonyle (2,3 millimoles de cobalt), 0,6 g de (pyridyl-2)-3-propanol (4,4 millimoles), 0,75 mL de tétrafluoro-borate de butyl-méthyl-imidazolium, 30 mL d'heptane et 30 mL d'hexène-1. On porte la pression du mélange hydrogène-monoxyde de carbone (1/1 molaire) à 6,5 MPa et la température à 95 °C et on met l'agitation en route. Après 6 heures, on arrête l'agitation et on laisse refroidir et décanter le mélange réactionnel, puis on relâche la pression. Après soutirage hors de l'autoclave, la phase organique supérieure est peu colorée. La conversion de l'hexène-1 est de 58 % en poids. La sélectivité pour les aldéhydes en C7 est de 92,8 % et le rapport n/iso (n-heptanal / isoheptanals) est égal à 3,9.

### EXEMPLE 7

La réaction d'hydroformylation est conduite dans le même appareillage et selon le même mode opératoire que décrit dans l'exemple 1. On introduit 0,4 g de dicobalt-octacarbonyle (2,3 millimoles de cobalt), 0,72 g de (pyridyl-2)-3-propyl-acétate (4 millimoles), 9 mL de bis-(trifluorométhyl-sulfonyl)-amidure de butyl-méthyl-imidazolium, 30 mL d'heptane et 30 mL d'hexène-1. On porte la pression du mélange hydrogène-monoxyde de carbone (1/1 molaire) à 9 MPa et la température à 95 °C et on met l'agitation en route. Après 6 heures, on arrête l'agitation et on laisse refroidir et décanter le mélange réactionnel, puis on relâche la pression. Après soutirage hors de l'autoclave, la phase organique supérieure est très légèrement colorée en jaune. La conversion de l'hexène-1 est de 86 % en poids. La sélectivité pour les aldéhydes en C7 est de 84,6 % et le rapport n/iso (n-heptanal / isoheptanals) est égal à 2,8.

### EXEMPLE 8

La réaction d'hydroformylation est conduite dans le même appareillage et selon le même mode opératoire que décrit dans l'exemple 1. On introduit 0,4 g de dicobalt-octacarbonyle (2,3 millimoles de cobalt), 0,8 g de N,N'-bis-(diisopropyl-2,6-phényl)-butane-2,3-diimine (2 millimoles), 6 mL de tétrafluoro-borate de butyl-méthyl-imidazolium, 30 mL d'heptane et 30 mL d'hexène-1. On porte la pression du mélange hydrogène-monoxyde de carbone (1/1 molaire) à 9,4 MPa et la température à 90 °C et on met l'agitation en route. Après 6 heures, on arrête l'agitation et on laisse refroidir et décanter le mélange réactionnel, puis on relâche la pression. Après soutirage hors de l'autoclave, la phase organique supérieure est peu colorée. La conversion de l'hexène-1 est de 95,5 % en poids. La sélectivité pour les aldéhydes en C7 est de 89,3 % et le rapport n/iso (n-heptanal / isoheptanals) est égal à 3.

### EXEMPLE 9

La réaction d'hydroformylation est conduite dans le même appareillage et selon le même mode opératoire que décrit dans l'exemple 1. On introduit 0,4 g de dicobalt-octacarbonyle (2,3 millimoles de cobalt), 0,72 g de (pyridyl-2)-3-propyl-acétate (4 millimoles), 6 mL de tétrafluoro-borate de tétra-(hexyl-octyl)-phosphonium, 30 mL d'heptane et 30 mL d'hexène-1. On porte la pression du mélange hydrogène-monoxyde de carbone (1/1 molaire) à 8,1 MPa et la température à 95 °C et on met l'agitation en route. Après 6 heures, on arrête l'agitation et on laisse refroidir et décanter le mélange réactionnel, puis on relâche la pression. Après soutirage hors de l'autoclave, la phase organique supérieure est peu colorée. La conversion de l'hexène-1 est de 57 % en poids. La sélectivité pour les aldéhydes en C7 est de 80 % et le rapport n/iso (n-heptanal / isoheptanals) est égal à 2,4.

### EXEMPLE 10 (comparatif)

La réaction d'hydroformylation est conduite dans le même appareillage et selon le même mode opératoire que décrit dans l'exemple 1. On introduit 0,35 g de dicobalt-octacarbonyle (2 millimoles de cobalt), 0,5 g de tri-n-butylphosphine (2,5 millimoles), 10 mL de tétrafluoro-borate de butyl-méthyl-imidazolium, 10 mL d'heptane et 30 mL d'hexène-1. On porte la pression du mélange hydrogène-monoxyde de carbone (1/1 molaire) à 10 MPa et la température à 125 °C et on met l'agitation en route. Après 6 heures, on arrête l'agitation et on laisse refroidir et décanter le mélange réactionnel, puis on relâche la pression. Après soutirage hors de l'autoclave, la phase organique supérieure est peu colorée. La conversion de l'hexène-1 est de 21 % en poids. La sélectivité pour les aldéhydes en C7 est de 34 % et le rapport n/iso (n-heptanal / isoheptanals) est égal à 2,7. La comparaison avec l'exemple 1 montre l'effet bénéfique d'un ligand azoté par rapport aux ligands phosphorés de l'art antérieur.

### EXEMPLE 11

La réaction d'hydroformylation est conduite dans le même appareillage et selon le même mode opératoire que décrit dans l'exemple 1. On introduit 19,3 mg d'acétylacétonate de rhodium-dicarbonyle, 30,3 mg de N,N'-bis-(diisopropyl-2,6-phényl)-butane-2,3-diimine, 5 g de tétrafluoroborate de tributyl-tétradécyl-phosphonium, 2 mL d'heptane et 7,5 mL d'hexène-1. On porte la pression du mélange hydrogène-monoxyde de carbone (1/1 molaire) à 2 MPa et la température à 80 °C et on met l'agitation en route. Après 5 heures, on arrête l'agitation et on laisse refroidir et décanter le mélange réactionnel, puis on relâche la pression. Après soutirage hors de l'autoclave, la phase organique supérieure est peu colorée. La conversion de l'hexène-1 est de 48 % en poids. La sélectivité pour les aldéhydes en C7 est de 60 % et le rapport n/iso (n-heptanal / isoheptanals) est égal à 2,4.

### EXEMPLE 12

La réaction d'hydroformylation est conduite dans le même appareillage et selon le même mode opératoire que décrit dans l'exemple 1. On introduit 19,3 mg d'acétylacétonate de rhodium-dicarbonyle, 18,3 mg d'acide 2,2'-bipyridyl-4,4'-dicarboxylique, 4 mL de tétrafluoro-borate de butyl-méthyl-imidazolium, 2 mL d'heptane et 7,5 mL d'hexène-1. On porte la pression du mélange hydrogène-monoxyde de carbone (1/1 molaire) à 2 MPa et la température à 80 °C et on met l'agitation en route. Après 5 heures, on arrête l'agitation et on laisse refroidir et décanter le mélange réactionnel, puis on relâche la pression. Après soutirage hors de l'autoclave, la phase organique supérieure est peu colorée. La conversion de l'hexène-1 est de 75 % en poids. La sélectivité pour les aldéhydes en C7 est de 66 % et le rapport n/iso (n-heptanal / isoheptanals) est égal à 1,2.

## Revendications

1. Procédé pour l'hydroformylation en phase liquide des composés oléfiniquement insaturés dans lequel la réaction est effectuée en présence d'une composition catalytique choisie parmi :
- celles qui comprennent au moins un complexe du cobalt coordiné par au moins un ligand azoté et au moins un solvant ionique non-aqueux comprenant au moins un sel organique-inorganique de formule générale Q⁺A⁻, dans laquelle Q⁺ représente un ammonium quaternaire et/ou un phosphonium quaternaire, et A⁻ représente tout anion susceptible de former un sel liquide en dessous de 90°C ; et
- celles qui comprennent au moins un complexe du rhodium coordiné par au moins un ligand azoté choisi dans le groupe formé par les imines, les diimines, la pyridine et les pyridines substituées, la bipyridine, l'imidazole et les imidazoles substitués, le pyrrole et les pyrroles substitués, le pyrazole et les pyrazoles substitués et au moins un solvant ionique non-aqueux comprenant au moins un sel organique-inorganique de formule générale Q⁺A⁻, dans laquelle Q⁺ représente un ammonium quaternaire et/ou un phosphonium quaternaire, et A⁻ représente tout anion susceptible de former un sel liquide en dessous de 90 °C.

2. Procédé selon la revendication 1 **caractérisé en ce que** les composés du cobalt et/ou du rhodium précurseurs du catalyseur sont choisis dans le groupe formé par les sels de cobalt et/ou du rhodium et les complexes carbonyles.

3. Procédé selon la revendication 1 **caractérisé en ce que** les composés du cobalt et/ou du rhodium précurseurs du catalyseur sont choisis dans le groupe formé par les acétylacétonates, les carboxylates, le dicobalt-octacarbonyle, l'hydrure de cobalt-tétracarbonyle, l'acétylacétonate de rhodium-dicarbonyle et les clusters carbonyles.

4. Procédé selon l'une des revendications 1 à 3 **caractérisé en ce que** ladite composition catalytique contient un complexe de cobalt et le ligand azoté est choisi dans le groupe formé par les mono-amines, les di-, tri- et polyamines, les imines, les diimines, la pyridine et les pyridines substituées, la bipyridine, l'imidazole et les imidazoles substitués, le pyrrole et les pyrroles substitués, le pyrazole et les pyrazoles substitués.

5. Procédé selon la revendication 4 **caractérisé en ce que** le ligand azoté est la pyridine ou une pyridine substituée.

6. Procédé selon la revendication 4 **caractérisé en ce que** le ligand azoté est l'imidazole ou un imidazole substitué.

7. Procédé selon la revendication 4 **caractérisé en ce que** le ligand azoté est une imine ou une diimine.

8. Procédé selon la revendication 7 **caractérisé en ce que** les anions A- sont choisis parmi les ions acétate, halogéno-acétates, tétrafluoro-borate, tétrachoro-borate, hexafluoro-phosphate, hexafluoro-antimonate, fluoro-sulfonate, perfluoroalkyl-sulfonates, bis-(perfluoroalkyl-sulfonyl)-amidures et arène-sulfonates.

9. Procédé selon la revendication 7 ou 8 **caractérisé en ce que** les ammonium et/ou phosphonium quaternaires répondent de préférence aux formules générales :
NR¹R²R³R⁴⁺
et
PR¹R²R³R⁴⁺
ou aux formules générales :
R¹R²N= CR³R⁴⁺
et
R¹R²P= CR³R⁴⁺
dans lesquelles R¹, R², R³ et R⁴, identiques ou différents, représentent l'hydrogène ou un rteste hydrocarbyle de 1 à 12 atomes de carbone, un seul substituant pouvant représenter l'hydrogène.

10. Procédé selon l'une des revendications 7 à 9 **caractérisé en ce que** les ammonium et/ou phosphonium sont dérivés d'hétérocycles azotés et/ou phosphorés comportant 1, 2 ou 3 atomes d'azote et/ou de phosphore, dans lesquelles les cycles sont constitués de 4 à 10 atomes.

11. Procédé selon l'une des revendications 7 à 10 **caractérisé en ce que** l'ammonium ou le phosphonium quaternaire sont constitués par un cation de formule:
R¹R²⁺N = CR³-R⁵-R³C = N⁺R¹R²
et/ou
R¹R²⁺P = CR³-R⁵-R³C = P⁺R¹R²
dans laquelle R1, R2 et R3, identiques ou différents, représentent l'hydrogène ou un reste hydrocarbyle de 1 à 12 atomes de carbone et R5 représente un reste alkylène ou phénylène.

12. Procédé selon l'une des revendications 7 à 11 **caractérisé en ce que** les ammonium et/ou phosphonium sont choisis dans le groupe formé par le N-butylpyridinium, le N-éthylpyridinium, l'éthyl-3 méthyl-1 imidazolium, le butyl-3 méthyl-1 imidazolium, le diéthylpyrazolium, le pyridinium, le triméthylphényl-ammonium et le tétrabutylphosphonium.

13. Procédé selon l'une des revendications 7 à 12 **caractérisé en ce que** le solvant ionique non-aqueux est choisi dans le groupe formé par l'hexafluorophosphate de N-butyl-pyridinium, le tétrafluoroborate de N-éthyl-pyridinium, le tétrafluoroborate de tétrabutylphosphonium, le tétrafluoroborate de butyl-3 méthyl-1 imidazolium, l'hexafluoroantimonate de butyl-3 méthyl-1 imidazolium, l'hexafluorophosphate de butyl-3 méthyl-1 imidazolium, le trifluorométhylsulfonate de butyl-3 méthyl-1 imidazolium , le fluorosulfonate de pyridinium, l'hexafluorophosphate de triméthyl-phénylammonium.

14. Procédé selon l'une des revendications 1 à 13 **caractérisé en ce que** la concentration du complexe de cobalt et/ou de rhodium dans le solvant ionique liquide est comprise entre 0,1 moles et 5 moles par litre et le rapport molaire entre le ligand azoté et le composé du cobalt et/ou du rhodium est compris entre 0,1 et 100.

15. Procédé selon l'une des revendications 1 à 14 **caractérisé en ce que** les composés oléfiniquement insaturés susceptibles d'être hydroformylés sont choisis dans le groupe formé par les monooléfines, les dioléfines, les composés oléfiniques comportant un ou plusieurs hétéroatomes.

16. Procédé selon l'une des revendications 1 à 15 **caractérisé en ce que** la réaction d'hydroformylation est effectuée avec un rapport des pressions partielles de l'hydrogène au monoxyde de carbone de 10:1 à 1:10, à une température comprise entre 30 °C et 200 °C, sous une pression comprise entre 1 MPa et 20 MPa.

## Patentansprüche

1. Verfahren zur Hydroformylierung von ungesättigten olefinischen Verbindungen in flüssiger Phase, bei dem die Reaktion in Gegenwart einer katalytischen Verbindung durchgeführt wird, die ausgewählt ist aus:
- denen, die umfassen wenigstens einen Kobaltkomplex koordiniert durch wenigstens einen stickstoffhaltigen Liganden und wenigstens ein nichtwässriges ionisches Lösungsmittel, umfassend wenigstens ein organisches/nichtorganisches Salz der allgemeinen Formel Q⁺A⁻, bei dem Q⁺ ein quarternäres Ammonium und/oder ein quarternäres Phosphonium darstellt, und A⁻ jedes Anion darstellt, welches in der Lage ist, ein flüssiges Salz unterhalb von 90°C auszubilden; und
- denen, die umfassen wenigstens einen Rhodiumkomplex koordiniert durch wenigstens einen stickstoffhaltigen Liganden ausgewählt aus der Gruppe gebildet durch Imine, Diimine, Pyridin und substituierte Pyridine, Bipyridin, Imidazol und substituierte Imidazole, Pyrrol und substituierte Pyrrole, Pyrazol und substituierte Pyrazole, und wenigstens ein nichtwässriges ionisches Lösungsmittel, umfassend wenigstens ein organisches/nichtorganisches Salz der allgemeinen Formel Q⁺A⁻, bei dem Q⁺ ein quarternäres Ammonium und/oder ein quarternäres Phosphonium darstellt, und A⁻ jedes Anion darstellt, welches in der Lage ist, ein flüssiges Salz unterhalb von 90°C auszubilden.

2. Verfahren gemäß Anspruch 1, **dadurch gekennzeichnet, dass** die Kobalt- und/oder Rhodium-Vorläuferverbindungen des Katalysators aus der Gruppe gewählt sind, die gebildet wird durch die Salze des Kobalts und/oder des Rhodiums und den Carbonyl-Komplexen.

3. Verfahren gemäß Anspruch 1, **dadurch gekennzeichnet, dass** die Kobalt- und/oder Rhodium-Vorläuferverbindungen des Katalysators aus der Gruppe gewählt sind, die gebildet wird durch die Acetylacetonate, Carboxylate, Dikobalt-Octacarbonyle, Hydride der Kobalt-Tetracarbonyle, Acetylacetonate der Rhodium-Dicarbonyle und den Carbonyl-Clustern.

4. Verfahren gemäß einem der Ansprüche 1 bis 3, **dadurch gekennzeichnet, dass** die genannte katalytische Zusammensetzung ein Kobalt-Komplex enthält und das der stickstoffhaltige Ligand aus der Gruppe gewählt ist, die gebildet wird durch die Mono-Amine, Di-, Tri- und Polyamine, Imine, Diimine, Pyridin und substituierte Pyridine, Bipyridin, Imidazol und substituierte Imidazole, Pyrrol und den substituierten Pyrrolen, Pyrazol und den substituierten Pyrazolen.

5. Verfahren gemäß Anspruch 4, **dadurch gekennzeichnet, dass** der stickstoffhaltige Ligand Pyridin oder ein substituiertes Pyridin ist.

6. Verfahren gemäß Anspruch 4, **dadurch gekennzeichnet, dass** der stickstoffhaltige Ligand Imidazol oder ein substituiertes Imidazol ist.

7. Verfahren gemäß Anspruch 4, **dadurch gekennzeichnet, dass** der stickstoffhaltige Ligand ein Imin oder ein Diimin ist.

8. Verfahren gemäß Anspruch 7, **dadurch gekennzeichnet, dass** die A⁻-Anionen aus den Ionen gewählt sind: Acetat, Halogenacetate, Tetrafluoroborate, Tetrachloroborate, Hexafluorophosphate, Hexafluoroantimonate, Fluorosulfonate, Perfluoroalkylsulfonate, bis-(Perfluoro-alkylsulfonyl)-Amide und A-rensulfonate.

9. Verfahren gemäß Anspruch 7 oder 8, **dadurch gekennzeichnet, dass** quarternäres Ammonium oder Phosphonium vorzugsweise den allgemeinen Formeln entspricht:
NR¹R²R³R⁴⁺
und
PR¹R²R³R⁴⁺
oder den allgemeinen Formeln entspricht:
R¹R²N=CR³R⁴⁺
und
R¹R²P=CR³R⁴⁺
bei denen R¹, R², R³ und R⁴⁺ identisch oder unterschiedlich sein können und Wasserstoff oder ein Hydrocarbylrest mit 1 bis 12 Kohlenstoffatomen sind, wobei ein einzelner Substituent Wasserstoff sein kann.

10. Verfahren gemäß einem der Ansprüche 7 bis 9, **dadurch gekennzeichnet , dass** Ammonium und/oder Phosphonium Derivate stickstoffhaltiger oder phosphorhaltiger Heterozyklen mit 1, 2 oder 3 Stickstoff- und/oder Phosphoratomen sind, bei denen die Ringe aus 4 bis 10 Atomen bestehen.

11. Verfahren gemäß einem der Ansprüche 7 bis 10, **dadurch gekennzeichnet, dass** quarternäres Ammonium oder Phosphonium gebildet werden durch ein Kation der Formel:
R¹R²⁺N=CR³R⁵-R³C=N⁺R¹R²
und/oder
R¹R²⁺P=CR³R⁵-R³C=P⁺R¹R²
bei denen R¹, R², R³ und R⁴⁺ identisch oder unterschiedlich sein können und Wasserstoff oder ein Hydrocarbylrest mit 1 bis 12 Kohlenstoffatomen sind und R⁵ ein Alkylen- oder Phenylen-Rest darstellt.

12. Verfahren gemäß einem der Ansprüche 7 bis 11, **dadurch gekennzeichnet, dass** das Ammonium und/oder das Phosphonium aus der Gruppe gewählt sind, die gebildet wird durch N-Butylpyridin, N-Ethylpyridin, Ethyl-3-Methyl-1-Imidazolin, Butyl-3-Methyl-1-Imidazolin, Diethylpyrazolin, Pyridin, Trimethylphenyl-ammonium und Tetrabutylphosphonium.

13. Verfahren gemäß einem der Ansprüche 7 bis 12, **dadurch gekennzeichnet, dass** das nicht-wässrige ionische Lösungsmittel aus der Gruppe gewählt ist, die gebildet wird durch das Hexafluorophosphat des N-Butyl-Pyridins, das Tetrafluoroborat des N-Ethyl-Pyridins, das Tetrafluoroborat des etrabutylphosphoniums, das Tetrafluoroborat des Butyl-3-Methyl-1-Imidazolins, das Hexafluoroantimonat des 3-Methyl-1-Imidazolins, das Hexafluorophosphat des Butyl-3-Methyl-1-Imidazolins, das Trifluormethylsulfonat des Butyl-3-Methyl-1-Imidazolins, das Fluorosulfonat des Pyridins, das Hexafluorophosphat des Trimethyl-Phenylammoniums.

14. Verfahren gemäß einem der Ansprüche 1 bis 13, **dadurch gekennzeichnet, dass** die Konzentration des Kobalt- und/oder Rhodiumkomplexes in dem flüssigen ionischen Lösungsmittel zwischen 0,1 mol/l und 5 mol/l liegt und das Molverhältnis zwischen dem stickstoffhaltigen Liganden und der Kobalt- und/oder Rhodiumverbindung zwischen 0,1 und 100 beträgt.

15. Verfahren gemäß einem der Ansprüche 1 bis 14, **dadurch gekennzeichnet, dass** zur Hydroformylierung geeigneten ungesättigten olenifischen Verbindungen aus der Gruppe gewählt sind, die gebildet wird durch Monoolefine, Diolefine, olefinische Verbindungen enthaltend ein oder mehrere Heteroatome.

16. Verfahren gemäß einem der Ansprüche 1 bis 15, **dadurch gekennzeichnet, dass** die Hydroformulierungsreaktion mit einem Partialdruckverhältnis von Wasserstoff zu Kohlenmonoxid von 10:1 bis 1:10, bei einer Temperatur zwischen 30°C und 200°C, unter einem Druck zwischen 1 MPa und 20MPa durchgeführt wird.

## Claims

1. A process for hydroformylation of olefinically unsaturated compounds in the liquid phase in which the reaction is carried out in the presence of a catalyst composition selected from:
- the ones that comprise at least one complex of cobalt co-ordinated by at least one nitrogen-containing ligand and at least one non-aqueous ionic solvent comprising at least one organic-inorganic salt with general formula Q⁺A⁻, where Q⁺ represents a quaternary ammonium and/or a quaternary phosphonium and A⁻ represents any anion which can form a liquid salt below 90°C; and
- the ones that comprise at least one complex of rhodium co-ordinated by at least one nitrogen-containing ligand selected from the group formed by imines, diimines, pyridine and substituted pyridines, bipyridine, imidazole and substituted imidazoles, pyrrole and substituted pyrroles, pyrazole and substituted pyrazoles and at least one non-aqueous ionic solvent comprising at least one organic-inorganic salt with general formula Q⁺A⁻,
where Q⁺ represents a quaternary ammonium and/or a quaternary phosphonium and A⁻ represents any anion which can form a liquid salt below 90°C.

2. A process according to claim 1, **characterized in that** the cobalt and/or rhodium precursor compounds of the catalyst are selected from the group formed by cobalt and/or rhodium salts and carbonyl complexes.

3. A process according to claim 1, **characterized in that** the cobalt and/or rhodium precursor compounds of the catalyst are selected from the group formed by acetylacetonates, carboxylates, dicobalt-octacarbonyl, cobalt-tetracarbonyl hydride, rhodium-dicarbonyl acetylacetonate and carbonyl clusters.

4. A process according to any one of claims 1 to 3, **characterized in that** said catalyst composition comprises a complex of cobalt and the nitrogen-containing ligand is selected from the group formed by monoamines, di-, tri- and polyamines, imines, diimines, pyridine and substituted pyridines, bipyridine, imidazole and substituted imidazoles, pyrrole and substituted pyrroles and pyrazole and substituted pyrazoles.

5. A process according to claim 4, **characterized in that** the nitrogen-containing ligand is pyridine or a substituted pyridine.

6. A process according to claim 4, **characterized in that** the nitrogen-containing ligand is imidazole or a substituted imidazole.

7. A process according to claim 4, **characterized in that** the nitrogen-containing ligand is an imine or a diimine.

8. A process according to claim 7, **characterized in that** the anions A⁻ are selected from acetate, halogenoacetate, tetrafluoroborate, tetrachloroborate, hexafluorophosphate, hexafluoroantimonate, fluorosulphonate, perfluoro-alkylsulphonate, bis-(perfluoroalkylsulphonyl)amide and arene-sulphonate ions.

9. A process according to claim 7 or claim 8, **characterized in that** the quaternary ammonium and/or phosphonium ions preferably have general formulae:
NR¹R²R³R⁴⁺
and
P R¹R²R³R⁴⁺
or general formulae:
R¹R²N=C R³R⁴⁺
and
R¹R²P=C R³R⁴⁺
wherein R¹, R², R³, and R⁴, which may be identical or different, represent hydrogen or a hydrocarbyl residue containing 1 to 12 carbon atoms, whereby a single substituent represents hydrogen.

10. A process according to any one of claims 7 to 9, **characterized in that** the ammonium and/or phosphonium ion can also be a derivative of nitrogen-containing and/or phosphorus-containing heterocycles containing 1, 2 or 3 nitrogen and/or phosphorus atoms, in which the cycles are constituted by 4 to 10 atoms, preferably 5 to 6 atoms.

11. A process according to any one of claims 7 to 10, **characterized in that** the quaternary ammonium or phosphonium ion is constituted by a cation with formula:
R¹R²⁺N=CR³-R⁵-R³C=N⁺R¹R²
and/or
R¹R²+P=CR³-R⁵-R³C=P+R¹R²
wherein R¹, R² and R³, which may be identical or different, represent hydrogen or a hydrocarbyl residue containing 1 to 12 carbon atoms and R5 represents an alkylene or phenylene residue.

12. A process according to any one of claims 7 to 11, **characterized in that** the ammonium and/or phosphonium ions are selected from the group formed by N-butylpyridinium, N-ethylpyridinium, 3-ethyl-1-methylimidazolium, 3-butyl-1-methylimidazolium, diethylpyrazolium, pyridinium, trimethylphenyl ammonium and tetrabutyl-phosphonium.

13. A process according to any one of claims 7 to 12, **characterized in that** the non-aqueous ionic solvent is selected from the group formed by N-butylpyridnium hexafluorophosphate, N-ethylpyridinium tetrafluoroborate, tetrabutylphosphonium tetrafluoroborate, 3-butyl-1-methylimidazolium tetrafluoroborate, 3-butyl-1-methylimidazolium hexaluoroantimonate, 3-butyl-1-methylimidazolium hexafluorophosphate, 3-butyl-1-methylimidazolium trifluoro-methylsulphonate, pyridinium fluorosulphonate and trimethylphenylammonium hexafluorophosphate.

14. A process according to any one of claims 1 to 13, **characterized in that** the concentration of cobalt and/or rhodium complex in the liquid ionic solvent is in the range 0.1 moles to 5 moles per litre, and the mole ratio between the nitrogen-containing ligand and the cobalt and/or rhodium compound is in the range 0.1 to 100.

15. A process according to any one of claims 1 to 14, **characterized in that** the olefinically unsaturated compounds which can be hydroformylated are selected from the group formed by monoolefins, diolefins and olefinic compounds comprising one or more heteroatoms.

16. A process according to any one of claims 1 to 15, **characterized in that** the hydroformylation reaction is carried out with the ratio of the partial pressures of hydrogen to carbon monoxide of 10:1 to 1:10, at a temperature in the range 30°C to 200°C, with a pressure in the range 1 MPa to 20 MPa.
